# DEMANDE DE BREVET EUROPEEN

(11) **EP 1 522 577 A1**
(43) Date de publication de la demande: **13.04.2005**
(21) Numéro de dépôt: 04292334.2
(22) Date de dépôt: 30.09.2004
(51) Int. Cl.: C12M 1/18

(54) **Enceinte de traitement d'échantillons biologiques à élément d'encadrement amovible**

(30) Priorité: 08.10.2003 FR 0311768
(71) Demandeur: Jouan, 44800 Saint Herblain (FR)
(72) Inventeur: Mimaud, Laurent, 44300 Nantes (FR); Ollivier, Véronique, 44220 Coueron (FR)
(74) Mandataire: Jacobson, Claude

(57) **Abrégé**

Cette enceinte (1) comprend un corps (3) qui comprend lui-même :
- un bâti (7) délimitant intérieurement un espace (9) de traitement qui débouche à l'extérieur du bâti (7) par une ouverture (13),
- au moins un élément d'encadrement (25I, 25S) fixé de manière amovible sur le bâti (7) pour délimiter dans l'ouverture (13) au moins deux zones (49A, 49B, 49C) d'accès à des régions distinctes de l'espace de traitement (9).

Au moins deux portillons sont montés mobiles sur le bâti (7) pour obturer et libérer sélectivement les zones d'accès (49A, 49B, 49C).

Application, par exemple, aux incubateurs à CO₂.

## Description

La présente invention concerne une enceinte de traitement d'échantillons biologiques, du type comprenant :
- un corps qui comprend lui-même :
   ■ un bâti délimitant intérieurement un espace de traitement des échantillons, l'espace de traitement débouchant à l'extérieur du bâti par une ouverture ménagée dans une face du bâti,
   ■ au moins un élément d'encadrement fixé de manière amovible sur le bâti pour former au moins deux cadres, les cadres délimitant dans l'ouverture au moins deux zones d'accès à des régions distinctes de l'espace de traitement,
- au moins deux portillons montés mobiles sur le corps pour obturer et libérer sélectivement les zones d'accès.

L'invention s'applique en particulier aux incubateurs à CO₂.

Un tel incubateur comprend des moyens de création, dans son espace de traitement, d'une atmosphère à teneur en CO₂, à humidité et à température contrôlée. De tels incubateurs sont par exemple utilisés pour la culture de cellules présentes dans des échantillons biologiques prélevés sur des patients.

L'espace de traitement comprend plusieurs régions délimitées par des étagères destinées à supporter des récipients contenant les échantillons biologiques.

Afin de limiter les perturbations induites dans l'atmosphère de l'espace de traitement lorsque l'on y accède, un tel incubateur comprend, outre sa porte principale généralement métallique, des portillons. Ces portillons, généralement réalisés en verre, permettent d'accéder sélectivement à chacune des régions de l'espace de traitement. Ainsi, pour accéder à une région déterminée de l'espace de traitement, on ouvre la porte principale puis uniquement le portillon correspondant à cette région.

Dans les incubateurs du type précité, l'élément d'encadrement est un ensemble métallique qui comprend tous les montants et toutes les traverses formant les cadres. Les portillons en verre sont articulés sur cet élément d'encadrement. L'élément d'encadrement est rapporté et fixé de manière libérable sur le bâti. Cette fixation libérable permet, en ôtant l'élément d'encadrement ainsi que les étagères de l'espace de traitement, de dégager complètement l'ouverture du bâti et l'espace de travail en vue de son nettoyage et de sa décontamination.

Toutefois, il existe un risque, lors du démontage de l'élément d'encadrement, de briser les portillons en verre portés par l'élément d'encadrement. En outre, l'encombrement et la masse de cet élément d'encadrement, rendent peu pratique l'opération de démontage et augmentent encore le risque de briser les portillons.

On connaît également des incubateurs dans lequel l'élément d'encadrement comprend, comme dans le cas précédent, tous les montants et toutes les traverses des cadres, mais est dans ce cas articulé sur le bâti. L'élément d'encadrement et les portillons articulés sur ce dernier forment alors une porte auxiliaire.

Pour accéder à une région de l'espace de traitement, on ouvre la porte principale puis le portillon correspondant. L'élément d'encadrement reste alors plaqué contre le bâti de l'incubateur.

Pour procéder au nettoyage à la décontamination, on ouvre la porte principale et la porte auxiliaire dans son ensemble, ce qui permet de dégager totalement l'accès à l'ouverture du bâti.

Cette structure permet de préparer plus simplement l'incubateur en vue du nettoyage et de la décontamination de son espace de travail, sans risque de briser les portillons. Toutefois, la porte auxiliaire s'avère complexe et coûteuse à réaliser, notamment pour pouvoir garantir sa fermeture étanche contre le bâti et pouvoir supporter la masse des portillons.

Un but de l'invention est de résoudre ces problèmes en fournissant une enceinte du type précité dont la structure soit simple et économique et qui limite les risques d'endommagement des portillons lors de la préparation de l'enceinte en vue de son nettoyage et de sa décontamination.

A cet effet, l'invention a pour objet une enceinte du type précité, caractérisée en ce que les portillons sont montés mobiles sur le bâti.

Selon des modes particuliers de réalisation, l'enceinte peut comprendre l'une ou plusieurs des caractéristiques suivantes, prise(s) isolément ou selon toutes les combinaisons techniquement possibles :
- l'élément d'encadrement est une traverse, et au moins un premier cadre est formé par la traverse et par le bâti ;
- l'élément d'encadrement comprend les deux cadres ;
- l'enceinte comprend des moyens d'étanchéité pour obturer de manière étanche les zones d'accès en s'étendant le long de leurs périphéries ;
- les moyens d'étanchéité sont montés sur les cadres ;
- les moyens d'étanchéité s'étendant le long de la périphérie de la zone délimitée par le premier cadre comprennent au moins un tronçon monté de manière amovible sur le bâti ;
- ledit tronçon est porté par la traverse grâce à laquelle il est monté de manière amovible sur le bâti ;
- le bâti comprend des moyens de détection de l'ouverture des portillons ;
- l'enceinte comprend en outre une porte principale articulée sur le bâti ; et
- elle constitue un incubateur.

L'invention sera mieux comprise à la lecture de la description qui va suivre, donnée uniquement à titre d'exemple, et faite en se référant aux dessins annexés, sur lesquels :
- la figure 1 est une vue schématique de face d'un incubateur à CO₂ selon l'invention, la porte principale étant en position ouverte et les portillons en positions fermées,
- la figure 2 est une vue analogue à la figure 1, les portillons étant en positions ouvertes,
- la figure 3 est une vue analogue à la figure 2, les éléments d'encadrement et les étagères ayant été retirés en vue du nettoyage et de la décontamination de l'espace de traitement ;
- la figure 4 est une schématique agrandie et en plan d'un élément d'encadrement de l'incubateur de la figure 1,
- les figures 5 et 6 sont des vues partielles, schématiques et en perspective illustrant la liaison des extrémités latérales d'un élément d'encadrement au bâti de l'incubateur à CO₂ de la figure 1, et
- la figure 7 est une vue schématique en plan d'un élément d'encadrement selon une variante de l'invention.

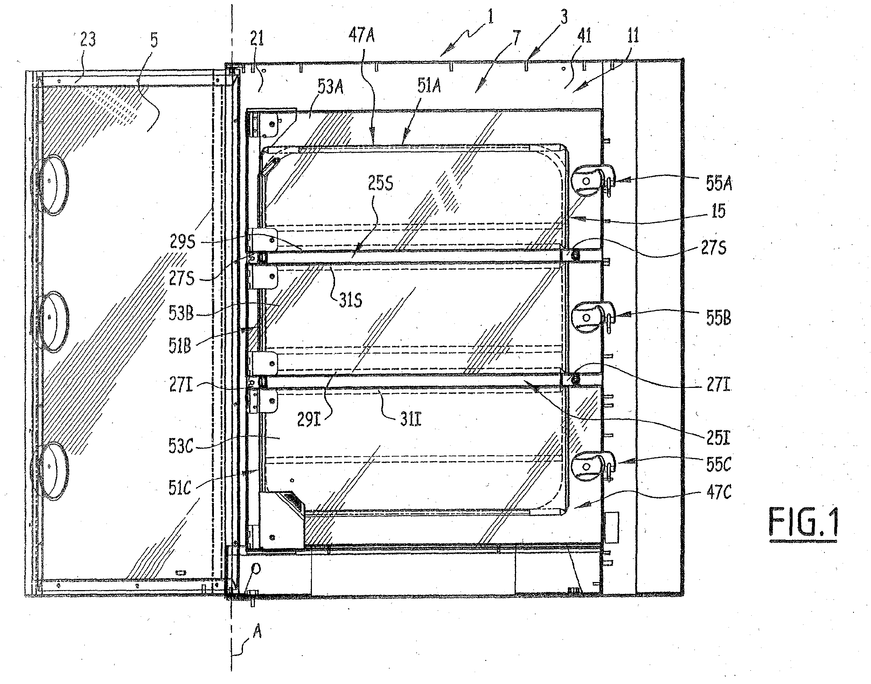

La figure 1 illustre schématiquement un incubateur à CO₂ 1 qui comprend un corps 3 et une porte principale 5.

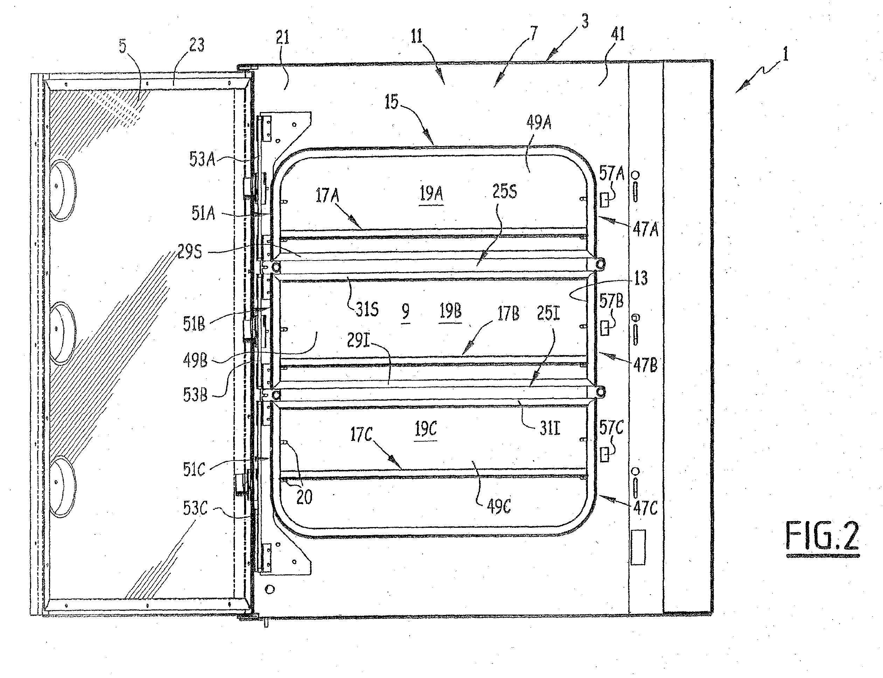

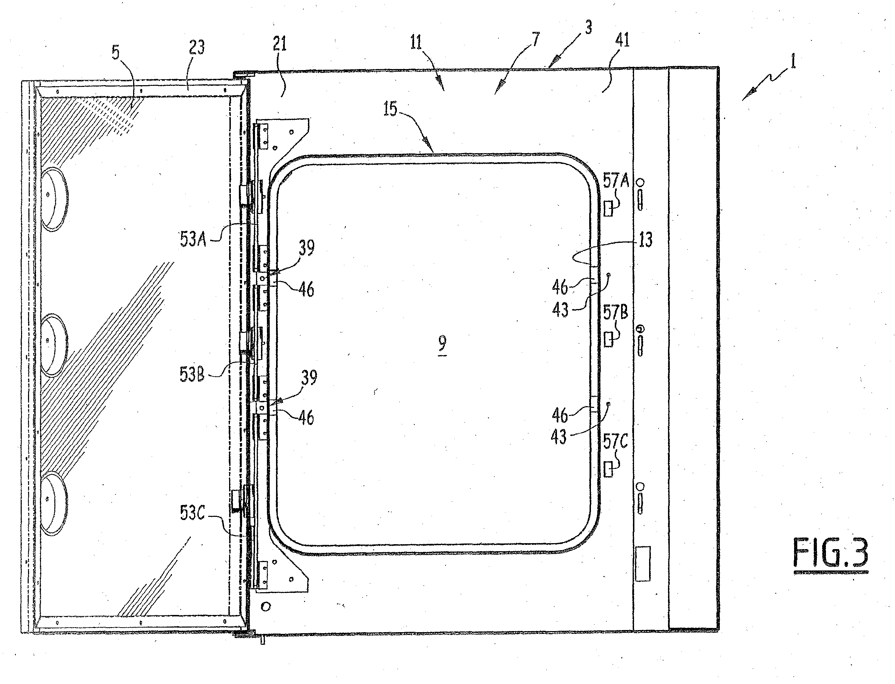

Le corps 3 comprend un bâti métallique 7 de forme générale parallélépipédique. Le bâti 7 délimite intérieurement un espace 9 de traitement d'échantillons biologiques, plus particulièrement visible sur les figures 2 et 3.

Pour mettre en oeuvre ce traitement, l'incubateur 1 comprend de manière classique des moyens de création dans l'espace 9 d'une atmosphère contrôlée. Cette atmosphère a ainsi une teneur en CO₂, une humidité et une température contrôlées.

L'espace de traitement 9 débouche dans la face avant 11 du bâti 7 par une ouverture 13 plus particulièrement visible sur les figures 2 et 3. Cette ouverture 13 a une forme générale rectangulaire à coins arrondis. La face avant 11 est munie d'un joint d'étanchéité 15 qui s'étend sur toute la périphérie de l'ouverture 13.

Comme on le voit sur la figure 2, l'incubateur 1 comprend trois étagères 17A, 17B et 17C qui sont disposées l'une au-dessus de l'autre de manière amovible à l'intérieur de l'espace de traitement 9. Ces trois étagères 17A, 17B et 17C délimitent ainsi dans l'espace de traitement 9 trois régions distinctes 19A, 19B et 19C disposées l'une au-dessus de l'autre.

Les étagères 17A, 17B et 17C sont par exemple simplement posées sur des barres d'appui 20 (figure 2) prévues sur les faces latérales de l'espace de traitement 9. Les barres d'appui 20 sont elles-mêmes amovibles:

La porte 5 est articulée au côté gauche 21 de la face 11. Ainsi, la porte 5 est mobile entre une position fermée (non-représentée), dans laquelle elle est plaquée contre la face avant 11, et une position ouverte illustrée par les figures 1 à 3, dans laquelle la porte 5 est éloignée de la face avant 11. Dans sa position fermée, un joint 23 prévu sur la porte 5 assure l'étanchéité entre le bâti 7 et la porte 5. La porte 5 empêche alors l'accès à l'ouverture 13.

Dans sa position ouverte, la porte 5 libère l'accès à cette ouverture 13.

De manière classique également, l'incubateur 1 peut comprendre des moyens de verrouillage magnétique de la porte 5 dans sa position fermée.

Le corps 3 de l'incubateur 1 comprend, outre le bâti 7, deux éléments d'encadrement 25S et 25I disposés l'un au-dessus de l'autre. Ces éléments d'encadrement ont une structure analogue. Les mêmes références numériques seront donc utilisées pour décrire ces deux éléments, suivies respectivement de la lettre I pour l'élément d'encadrement inférieur et de la lettre S pour l'élément d'encadrement supérieur.

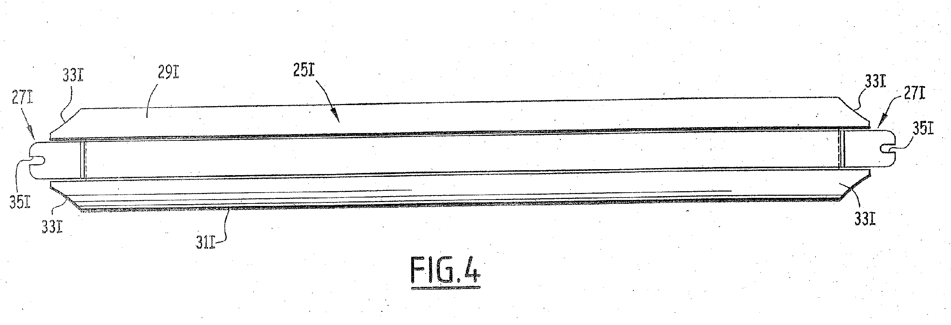

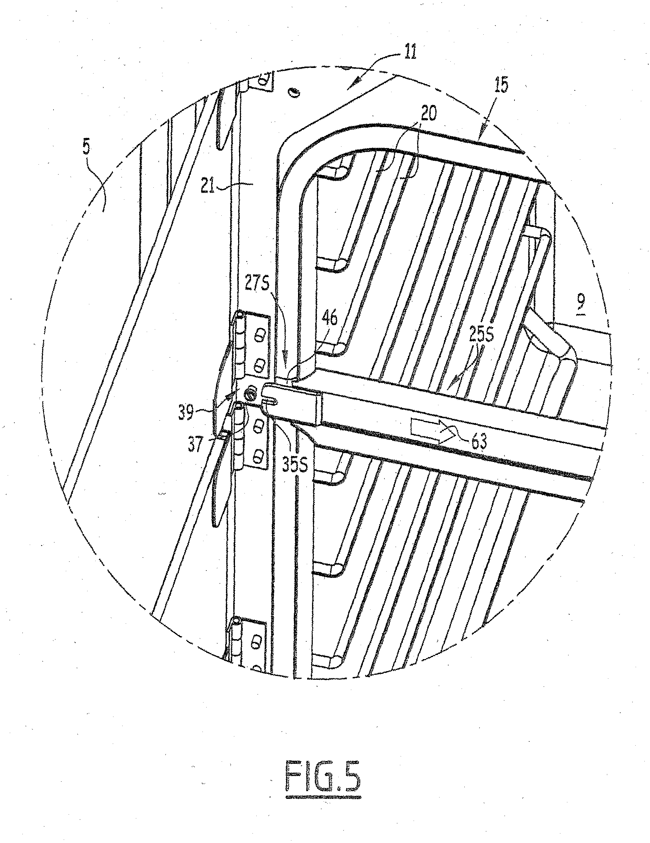

Comme on le voit sur la figure 4, les éléments d'encadrement 25I et 25S sont des traverses métalliques dont les extrémités latérales 27I, 27S présentent des décrochements vers l'avant. Ainsi, les parties centrales des éléments 25I, 25S sont en retrait par rapport aux extrémités 27I, 27S, comme on le voit par exemple sur la figure 5. Les éléments d'encadrements 251, 25S sont en outre munis de joints d'étanchéité rectilignes 291, 29S qui s'étendent le long de leurs bords supérieurs et de joints d'étanchéité rectilignes 31I, 31S qui s'étendent le long de leurs bords inférieurs. Ces joints d'étanchéité comprennent des lèvres d'extrémité 33I, 33S qui sont biseautées et convergent vers les extrémités latérales respectives 271, 27S.

Les extrémités latérales 271, 27S présentent des encoches 35I, 35S permettant la fixation amovible des éléments d'encadrement 251 et 25S sur le bâti 7.

Plus précisément et comme on le voit sur la figure 5, les extrémités gauches 27I, 27S sont destinées à s'engager derrière des cols 37 de deux pions de retenue 39 solidaires du côté gauche 21 de la face avant 11. Les pions 39 sont alors reçus dans les encoches 351, 35S gauches et les extrémités gauches 27I, 27S sont plaquées contre le bâti 7.

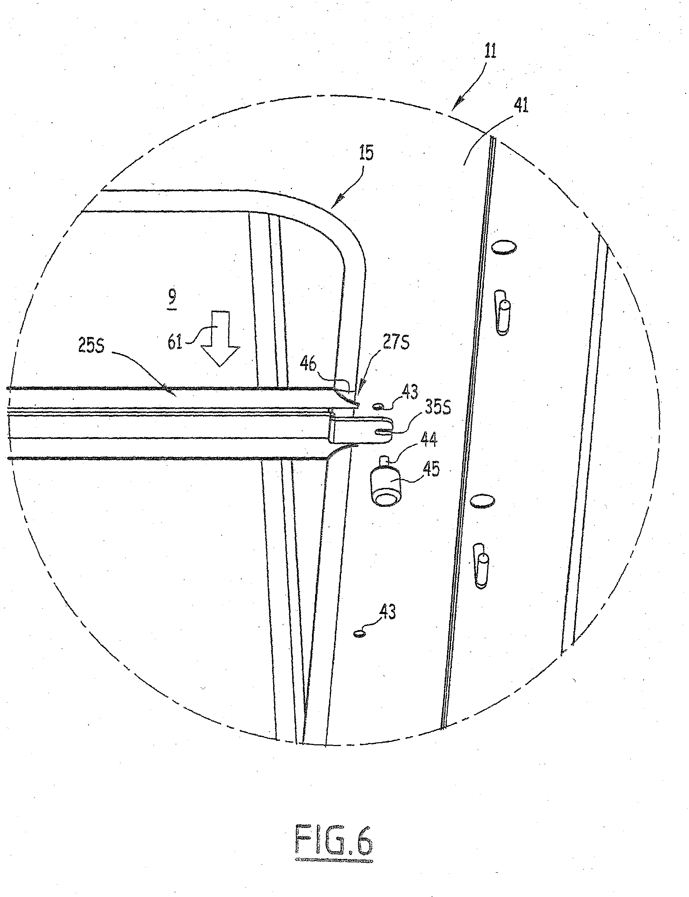

Comme illustré par la figure 6, le côté droit 41 de la face avant 11 du bâti 7 comporte deux orifices taraudés 43 destinés à recevoir des vis 44, prévues sur deux boutons moletés 45. Les vis 44 s'engagent alors dans les encoches 35I, 35S droites des éléments d'encadrement 251 et 25S.

Les boutons moletés 45 plaquent alors les extrémités droites 27I et 27S des éléments d'encadrement 25I, 25S contre la face avant 11.

On notera que le joint 15 présente des décrochements 46 permettant le passage des extrémités 27I et 27S des éléments d'encadrement 15. En variante, ces décrochements 46 peuvent être remplacés par des interruptions du joint 15.

Les éléments d'encadrement 251 et 25S sont alors solidaires du bâti 7 et s'étendent transversalement au travers de l'ouverture 13. Les éléments d'encadrement 251 et 25S forment alors avec le bâti trois cadres 47A, 47B et 47C, comme on le voit sur la figure 2.

La traverse inférieure du cadre supérieur 47A est formée par l'élément d'encadrement 25S et le reste du cadre 47A, à savoir ses montants latéraux et sa traverse supérieure sont formés par la face avant 11 du bâti 7.

Les traverses horizontales du cadre intermédiaire 47B sont formées par les éléments d'encadrement 25I et 25S. Les deux montants latéraux du cadre 47B sont formés par la face avant 11 du bâti 7.

La traverse supérieure du cadre inférieur 47C est formée par l'élément d'encadrement 251. Le reste de ce cadre 47C est formé par la face avant 11 du bâti 7.

Comme on le voit sur la figure 2, les cadres 47A, 47B, 47C délimitent dans l'ouverture 13 trois zones 49A, 49B et 49C, qui sont disposées l'une au-dessus de l'autre, et qui permettent respectivement d'accéder aux régions 19A, 19B et 19C de l'espace de traitement 9.

Les lèvres 331 et 33S des joints 29I, 29S, 31I et 31 S viennent recouvrir le joint 15 et épouser sa forme. Ainsi, les ouvertures 49A, 49B et 49C sont entourées par des moyens d'étanchéité respectivement référencés 51A, 51B et 51 C.

Les moyens 51A d'étanchéité comprennent un tronçon supérieur et deux tronçons latéraux formés par le joint d'étanchéité 15. Le tronçon inférieur des moyens 51A d'étanchéité est formé par le joint supérieur 29S de l'élément d'encadrement supérieur 25S.

Les moyens 51B d'étanchéité comprennent deux tronçons horizontaux formés par les joints 29I et 31S et deux tronçons latéraux formés par le joint 15.

Les moyens 51 C d'étanchéité comprennent un tronçon supérieur formé par le joint 31I et deux tronçons latéraux et un tronçon inférieur formé par le joint 15.

Grâce aux décrochements des extrémités latérales 27I et 27S des éléments d'encadrement 251 et 25S, les tronçons formés par les joints 15, 29I, 29S, 31I et 31 S sont situés sensiblement dans un même plan. Ainsi, tous les tronçons des moyens 51A, 51B et 51C d'étanchéité sont situés respectivement dans un même plan.

Comme on le voit sur la figure 1, l'incubateur 1 comprend en outre trois portillons 53A, 53B et 53C articulés au côté gauche 21 de la face 11 par des charnières pour pouvoir être mobiles par rapport au bâti 7 indépendamment l'un de l'autre. Les portillons 53A, 53B et 53C sont par exemple réalisés en verre, et

sont plus généralement transparents pour pouvoir discerner depuis l'extérieur le contenu de l'espace de traitement 9.

Les portillons 53A, 53B et 53C sont mobiles par rapport au bâti 7 entre des positions fermées, dans lesquelles ils obturent respectivement les zones 49A, 49B et 49C de l'ouverture 13 et sont plaquées contre les moyens 51A, 51B et 51 C d'étanchéité, et des positions ouvertes de libération des zones 49A, 49B et 49C.

Les positions fermées sont illustrées par la figure 1 et les positions ouvertes par les figures 2 et 3.

Dans les positions fermées, les différents joints 15, 291, 29S, 31I et 31S sont écrasés et les moyens d'étanchéité 51A, 51B, 51C assurent donc l'étanchéité entre la face avant 11 du bâti 7 et les portillons 53A, 53B et 53C.

Les côtés droits des portillons 53A, 53B et 53C sont munis de moyens 55A, 55B et 55C de verrouillage des portillons dans leurs positions fermées.

En outre, le bâti 7 peut comprendre sur le côté droit 41 de sa face avant 11, des capteurs 57A, 57B et 57C d'ouverture des portillons 53A, 53B et 53C. Il peut s'agir d'interrupteurs qui sont reliés électriquement au système de commande et de contrôle de l'incubateur 1.

Pour accéder à une des régions 19A, 19B et 19C de l'espace de traitement 9 lors d'un fonctionnement normal de l'incubateur 1, on ouvre successivement la porte 5 puis le portillon 53A, 53B ou 53C correspondant. On effectue alors l'opération souhaitée dans la région choisie de l'espace de traitement 9. Il peut s'agir par exemple de l'introduction ou du retrait de récipients contenant des échantillons. Un seul des portillons 53A, 53B et 53C étant alors ouvert, les perturbations induites dans l'atmosphère de l'espace de traitement 9 sont réduites. A l'issue de l'opération, on referme le portillon choisi et la porte 5.

Pour pouvoir procéder au nettoyage et à la décontamination de l'espace de traitement 9, on ouvre tout d'abord la porte 5 et tous les portillons 53A, 53B et 53C, comme illustré par la figure 2.

On retire ensuite les éléments d'encadrement 25I et 25S. Cela peut être assuré en dévissant les boutons 45 puis en écartant les extrémités droites 27I et 27S des éléments 251 et 25S du côté droit 41 de la face avant 11, comme matérialisé par la flèche 61 sur la figure 6, puis en tirant les éléments d'encadrement 25I et 25S vers la droite afin de dégager leurs extrémités gauches 27I et 27S du bâti 7, comme illustré par la flèche 63 sur la figure 5. Enfin, on retire les étagères 17A, 17B et 17C et les barres d'appui 20.

L'incubateur 1 est alors dans la configuration de la figure 3. L'ouverture 13 et l'espace de traitement 9 sont entièrement dégagés, permettant ainsi un accès total aux parois de l'espace 9. Le nettoyage et la décontamination peuvent alors être effectués.

Pour utiliser à nouveau l'incubateur 1, on procède à l'inverse de ce qui a été décrit précédemment, notamment en engageant tout d'abord les extrémités gauches 27I, 27S des éléments d'encadrement 25I et 25S derrière les collerettes 37 puis en venant fixer les extrémités droites 271 et 27S par vissage des boutons 45.

Ainsi, les éléments d'encadrements 251 et 25S sont légers et peuvent être enlevés très facilement pour le nettoyage et la décontamination de l'incubateur 1.

En outre, le risque de briser les portillons 53A , 53B et 53C est réduit, puisqu'ils sont montés sur le bâti 7, et non sur les éléments d'encadrement comme dans l'état de la technique, et ne sont donc pas démontés lors de l'enlèvement des éléments 251 et 25S en vue du nettoyage et de la décontamination. En outre, la structure des éléments d'encadrement 25I et 25S permet de conserver une structure globale simple et un coût réduit pour l'incubateur 1.

On observera également que les éléments d'encadrement 25I et 25S permettent de disposer de capteurs 57A, 57B et 57C de détection de l'ouverture des portillons 53A, 53B et 53C sans induire de problème de raccordement de ces capteurs au système de commande et de contrôle de l'incubateur 1, puisque ces capteurs sont prévus sur le bâti 7 lui-même.

Les moyens 51A, 51 B et 51 C d'étanchéité peuvent non pas être réalisés par des tronçons fixes par rapport au bâti 7 mais comprendre au moins des tronçons amovibles. Il peut ainsi s'agir d'éléments continus entièrement amovibles que l'on retire pour le nettoyage et pour la décontamination. En variante également, les moyens d'étanchéité 51A à 51C peuvent être prévus sur les portillons eux-mêmes, plutôt que sur le corps 3. On peut également envisager de ne pas utiliser de moyens 51A, 51 B et 51C d'étanchéité.

De manière plus générale, les éléments d'encadrement peuvent avoir une forme différente associée à une configuration différente des cadres formés. Ainsi, un élément d'encadrement distinct peut être utilisé pour chaque cadre, plutôt que des traverses communes à plusieurs cadres comme dans l'exemple précédent.

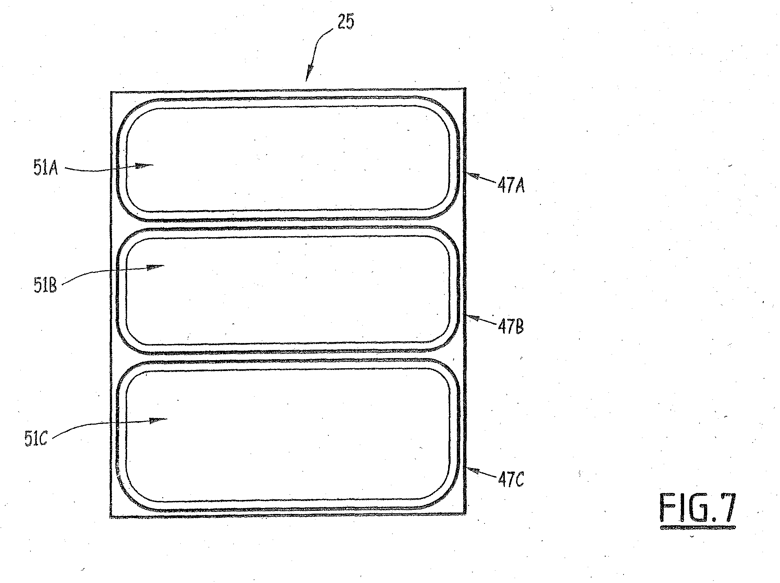

Comme illustré par la figure 7, on peut également utiliser un élément d'encadrement 25 complet, c'est à dire comprenant l'ensemble des cadres 47A, 478, 47C. Toutefois, les portillons 53A, 53B, 53C restent alors montés sur le bâti 7 plutôt que sur cet élément d'encadrement 25 afin de conserver une structure simple et de limiter les risques d'endommagement des portillons, lors de la préparation de l'incubateur 1 en vue de son nettoyage et de sa décontamination.

De manière plus générale également, le nombre de cadres 47 formés et donc de portillons 53 peut être différent de celui décrit précédemment.

L'invention peut également s'appliquer à tout type d'enceinte de traitement d'échantillons biologiques. On entend par traitement la soumission des échantillons à une atmosphère contrôlée, par exemple en température. Ce terme s'étend donc également au stockage. Ainsi, l'invention peut s'appliquer notamment aux étuves, aux réfrigérateurs...

## Revendications

1. Enceinte (1) de traitement d'échantillons biologiques, du type comprenant :
- un corps (3) qui comprend lui-même
■ un bâti (7) délimitant intérieurement un espace (9) de traitement des échantillons, l'espace de traitement (9) débouchant à l'extérieur du bâti (7) par une ouverture (13) ménagée dans une face (11) du bâti,
■ au moins un élément d'encadrement (25I, 25S ; 25) fixé de manière amovible sur le bâti (7) pour former au moins deux cadres (47A, 47B, 47C), les cadres délimitant dans l'ouverture (13) au moins deux zones (49A, 49B, 49C) d'accès à des régions distinctes (19A, 19B, 19C) de l'espace de traitement (9),
- au moins deux portillons (53A, 53B, 53C) montés mobiles sur le corps (3) pour obturer et libérer sélectivement les zones d'accès (49A, 49B, 49C),
**caractérisée en ce que** les portillons (53A, 53B, 53C) sont montés mobiles sur le bâti (7).

2. Enceinte selon la revendication 1, **caractérisée en ce que** l'élément d'encadrement (25I, 25S) est une traverse, et **en ce qu'**au moins un premier cadre (47A, 47B, 47C) est formé par la traverse et par le bâti (7).

3. Enceinte selon la revendication 1, **caractérisée en ce que** l'élément d'encadrement (25) comprend les deux cadres (47A, 47B, 47C).

4. Enceinte selon l'une des revendications précédentes, **caractérisée en ce qu'**elle comprend des moyens d'étanchéité (51A, 51B, 51C) pour obturer de manière étanche les zones d'accès (49A, 49B, 49C) en s'étendant le long de leurs périphéries.

5. Enceinte selon la revendication 4, **caractérisée en ce que** les moyens (51A, 51B, 51C) d'étanchéité sont montés sur les cadres (47A, 47B, 47C).

6. Enceinte selon la revendication 5 lorsqu'elle dépend de la revendication 2, **caractérisée en ce que** les moyens d'étanchéité s'étendant le long de la périphérie de la zone (49A, 49B, 49C) délimitée par le premier cadre (47A, 47B, 47C) comprennent au moins un tronçon (29I, 29S, 31I, 31S) monté de manière amovible sur le bâti (7).

7. Enceinte selon la revendication 6, **caractérisée en ce que** ledit tronçon (29I, 29S, 31I, 31 S) est porté par la traverse grâce à laquelle il est monté de manière amovible sur le bâti.

8. Enceinte selon l'une des revendications précédentes, **caractérisée en ce que** le bâti (7) comprend des moyens (57A, 57B, 57C) de détection de l'ouverture des portillons.

9. Enceinte selon l'une des revendications précédentes, **caractérisée en ce qu'**elle comprend en outre une porte principale (5) articulée sur le bâti (7).

10. Enceinte selon l'une des revendications précédentes, **caractérisée en ce qu'**elle constitue un incubateur.
